# NEW EUROPEAN PATENT SPECIFICATION

(11) **EP 0 223 590 B2**
(45) Date of publication and mention of the opposition decision: **11.09.1996**
(45) Mention of the grant of the patent: 07.07.1993
(21) Application number: 86308985.0
(22) Date of filing: 18.11.1986
(51) Int. Cl.: A61K 9/22

(54) **Substained release compositions comprising hydroxypropyl cellulose ethers**
Hydroxypropylcelluloseether enthaltende Mittel mit verzögerter Wirkstoffabgabe
Compositions à libération retardée contenant des éthers hydroxypropyl cellulosiques

(30) Priority: 18.11.1985 US 799044
(43) Date of publication of application: 27.05.1987
(73) Proprietor: THE DOW CHEMICAL COMPANY, Midland Michigan 48640-1967 (US)
(72) Inventor: Alderman, Daniel A., Midland Michigan 48640 (US)
(74) Representative: Burford, Anthony Frederick

(56) References cited:
- WO-A-84/00104
- GB-A- 1 362 700
- GB-A- 2 152 940
- JP-B- 5 924 964
- US-A- 3 852 421
- US-A- 4 091 205
- US-A- 4 259 314
- US-A- 4 389 393
- RESEARCH DISCLOSURE, no. 259, November 1985, pages 567-570, disclosure no. 25914, Emsworth, Hampshire, GB; "Sustained released compositions"
- CHEMICAL ABSTRACTS, vol. 92, no. 10, 10th March 1980, page 389, abstract no. 82425d, Columbus, Ohio, US; & JP-A-79 74 855 (TAKEDA CHEMICAL INDUSTRIES LTD) 15-06-1979
- Merck Index, 10th Ed., 1983, Mon. 4763 & 4764
- Martindale, Extra Pharmacopoeia, 28th Ed., 1982, Mon. 5431 & 5432
- The United States Pharmacopeia, NF XVI
- Pharmacopeia of Japan, p. D-730
- D.A. Alderman in Int. J. Pharm. Tech. & Prod. Mfr. 5(3) 1-9, 1984
- Remington's Pharmaceutical Sciences, 17th Ed. 1985
- The theory and practice of industrial pharmacy, Lea & Febiger, 1986

## Description

This invention relates to sustained release solid pharmaceutical formulations, particularly tablets, comprised of hydrophilic polymeric cellulose ether.

Polymeric compositions have been widely used as a matrix base for compressed tablets. Such tablets typically contain at least one medicament or vitamin whose rate of release into the system is delayed or controlled by the matrix base. Controlled release tablets are desirable because they provide a method of delivering a long-lasting dose in a single application without overdosing the patient.

Typically, an effective amount of the polymeric matrix composition is employed. It is desirable to employ as little amount of polymeric composition as possible to provide the intended release profile, to obtain minimum dosage size or to obtain good compression properties. For such applications, a highly hydrophilic polymeric composition is suitably employed. Such a composition rapidly hydrates and forms a gel-like layer in the tablet through which the active ingredient is released to the system. An example of a preferred hydrophilic polymeric composition is a cellulose ether sold as METHOCEL® K4M and K15M by The Dow Chemical Company, which has a hydroxypropoxyl substitution of between 4 to 12 weight percent, and a methoxyl substitution of between 19 to 25 weight percent.

US-A-4,369,172 discloses that hydroxypropyl methylcellulose ethers having a hydroxypropoxyl content of from 9 to 12 percent and a number average molecular weight of less than about 50,000 provide the best sustained release. Moreover, the effect of hydration and gel formation is deemphasized in favor of , the chemical composition of the hydroxypropyl methylcellulose.

Cellulose ethers, such as METHOCEL® K, are desirable polymeric matrix compositions because they are derived from naturally occurring cellulose, and are free-flowing, readily compressible powders. Unfortunately, not all cellulose ethers hydrate rapidly, and therefore do not provide a desirable release profile for compressed tablets. For example, hydroxypropyl cellulose is commercially available in granular form. Such granules are not suitable for compressing tablets and do not hydrate rapidly.

JP-A-79-74855 discloses the use of fine particle sized water-soluble cellulose derivatives as tablet binders. The powder is formed by spray-drying a solution of the cellulose derivative and has an average diameter of less than 20 micrometers, preferably less than 15 micrometers. In one example, tablets are formed by compressing granules containing 73 weight percent penicillin, 22.5 weight percent lactose, 4 weight percent the fine particle sized hydroxypropyl cellulose and 0.5 weight percent magnesium stearate. There is no teaching of the fine particle size causing any delay in release of active ingredient from tablets.

US-A-3852421 discloses the use of certain water-insoluble hydroxyalkyl and hydroxyalkyl alkyl celluloses as excipients to promote tablet disintegration. The cellulose derivatives are required to have an average total of 0.1 to 1.30 substituted moles of hydroxyalkyl groups and alkyl groups per anhydrous glucose units, 0.05 to 1 substituted moles of hydroxyalkyl group per anhydrous glucose unit and 0 to 1.0 average substituted moles alkyl groups per anhydrous glucose unit. It is preferred to use cellulose derivatives as particles of a fine size such that at least 90% are less than 100 micrometers, preferably less than 80 micrometers, in order to reduce disintegration time.

Research Disclosure No. 25914 discloses that release of an active ingredient from a sustained release tablet comprising an hydroxypropyl methylcellulose ether (HPMC) of specified grade is delayed by reducing the particle size of the cellulose ether. In particular, the cellulose ether is required to have a hydroxypropoxyl substitution of from about 7 to about 12 weight percent, a methoxyl substitution of from about 28 to about 30 weight percent and a number average molecular weight of at least 15,000. At least about 90 weight percent, preferably at least about 95 weight percent, of the particles of said cellulose ether are required to pass through a 100 mesh (150 micrometer) screen. There is no teaching that said particle size would delay tablet disintegration when using other cellulose ethers. On the contrary, since the prior art acknowledgement in this document refers to cellulose ethers having a hydroxypropoxyl substitution of from about 4 to about 12 weight percent and a methoxyl substitution of from about 19 to about 25 weight percent, it clearly teaches that hydroxypropoxyl and, especially, methoxyl substitution is critical to the prolonged sustained release. There is a reference to hydroxypropyl cellulose in a list of suitable tablet binders but no teaching that said binders should be used in any unconventional particle size.

Yet another factor affecting the performance of the tablet is the chemical characteristics of the active ingredient employed. Certain polymers can be employed beneficially for some active ingredients, but not for others. The active ingredient's degree of water-solubility, molecular weight, and the diffusion coefficient in a hydrated polymer gel layer can be critical.

It would be desirable to have additional cellulose ether polymeric matrix materials which would provide sufficient release profiles for use in pharmaceutical formulations such as particularly tablets.

The present invention provides a sustained release solid pharmaceutical formulation for oral administration comprising at lease one active ingredient; 5 to 90 weight percent of a polymeric matrix base comprising a water-soluble cellulose ether and, optionally, a hydrophilic colloid; and optionally one or more carriers or excipients, characterised in that said cellulose ether is hydroxypropyl cellulose ether having hydroxypropoxyl substitution of from 20 to 80 weight percent and of a fine particle size whereby at least 50 weight percent of said cellulose ether particles can pass through a 100 mesh (150 micrometers) screen.

The formulation is prepared by intimately mixing an effective amount of the active ingredient(s) in the form of a powder with a functionally effective amount of a fine particle sized hydroxypropyl cellulose ether. The cellulose ether is in the form of a powder. When present, the optional ingredients are also added, preferably as powders. The intimate mixture is subjected to compression conditions to form a solid tablet. The cellulose ether composition is sufficiently fine that the release of active composition from the solid tablet is delayed longer upon contacting an aqueous acidic environment at 37° C; compared to a tablet formulated with a chemically identical but coarser particle sized hydroxypropyl cellulose ether composition.

This invention is useful in providing solid pharmaceutical formulations having at least one therapeutically active ingredient with a cellulose ether which exhibits sustained release properties and optionally also has carriers and excipients.

The hydroxypropyl cellulose ether composition (HPC) of this invention has a hydroxypropoxyl substitution sufficient to render the polymer water-soluble. Such amount can vary and typically ranges from 20 to 80 weight percent, and preferably from 40 to 80 weight percent, as described in the National Formulary. Such cellulose ethers can be prepared by reacting propylene oxide with alkali cellulose. Hydroxypropyl cellulose ether is readily commercially available under the trade name KLUCEL® from Hercules, and NISSO HPC® from Nippon Soda.

The HPC of this invention is in the form of a powder and has a particle size sufficiently fine that the release of active ingredients from a solid formulation is delayed longer upon contacting an aqueous acidic environment at 37° C; compared to a tablet formulated with a chemically identical but coarser particle sized HPC. Typically, such particle size is sufficient that the HPC hydrates rapidly, forming a gel-like layer, upon contacting an aqueous environment. Such particle size can vary, although any size sufficient to form a gel-like layer can be employed, and typically is sufficient when at least 50 weight percent of the particles can pass through a 100 mesh (150 micrometers) screen, and preferably when at least 70 weight percent can pass through a 100 mesh (150 micrometers) screen.

The cellulose ether is chemically identical, for purposes of this invention, when it possesses hydroxypropoxyl substitution as defined as HPC NF grade and has a 2 percent aqueous solution viscosity within about 50 percent of the viscosity of the fine particle sized composition. The cellulose ether is coarse when the particle size distribution has a larger amount by weight of larger particles than the fine particle sized cellulose ether.

The cellulose ether is substantially water-soluble. Substantially water-soluble ethers tend to spontaneously disperse their molecules throughout the molecules of water.

A functionally effective amount of the cellulose ether is employed. Such amount is an amount sufficient to delay the release of the therapeutically active ingredient. Preferably, the amount employed is the minimum amount required to provide the delayed release. Such an amount can vary and typically ranges from 5 to 90 weight percent, preferably from 5 to 25 weight percent, and most preferably from 10 to 17 weight percent based on weight of the tablet, although any functionally effective amount can be employed.

The solid pharmaceutical formulation can be administered orally to affect a condition such as, for example, a pharmaceutical drug or vitamin. The active ingredient(s) can be a water-soluble or a water-insoluble composition. A water-soluble composition is a composition which spontaneously disperses its molecules in an aqueous medium, and a water-insoluble composition is a composition which does not exhibit that spontaneous dispersion. Suitable water-soluble compositions include, for example, aspirin, theophylline, pseudoephedrine HCl, ascorbic acid, riboflavin, and others. Suitable water-insoluble compositions include, for example, naproxyn, ibuprofen and others. Water-soluble compositions especially find the process of this invention useful because they tend to dissolve and diffuse through the hydrated cellulose ether layer during gel formation.

The therapeutically active ingredient(s) is employed in any effective dosage amount. Such amount is an amount sufficient to affect the condition to be treated. The amount can vary according to the specific active ingredient(s) employed, and such variations are within the knowledge of the skilled artisan. Typically, the active ingredient can be employed up to 95 weight percent of the compressed tablet, although any effective weight percent can be employed.

Optionally, an additional hydrophilic colloid can be employed in conjunction with the HPC of this invention. Suitable hydrophilic colloids are water-soluble cellulose ethers, such as hydroxypropyl methylcellulose (HPMC). It is not critical that such optional hydrophilic colloids be fine particle sized compositions, although for certain active ingredients it can be desirable. For such compositions, it is desirable that the HPMC have a particle size sufficient that at least 70 weight percent can pass through a 100 mesh (150 micrometers) screen and preferably at least 70 weight percent can pass through a 140 mesh (100 micrometers) screen. The HPMC's which are designated as HPMC 2208 USP and HPMC 2910 are preferred. Such HPMC's have a hydroxypropoxyl content of between 4 and 12 weight percent and a methoxyl content of between 19 and 30 weight percent. The HPMC's can exhibit a 2 percent aqueous solution viscosity of between 100 and 100,000 cps (0.1 - 100 Pa.s), and are readily commercially available from The Dow Chemical Company under the trade name METHOCEL®.

The optional HPMC can be employed in any functionally effective amount. Such amount can vary, and typically ranges from 5 to 75 weight percent of polymer mixture, and up to 90 weight percent of the tablet in combination with the HPC. Preferably, the HPC is employed in greater than 30 weight percent based on the combined weight percent of HPMC and HPC, because the flow properties and thereby the compression properties for tablet formation are improved.

Typically, tablets can contain one or more optional carriers or excipients such as diluents or fillers, binders, lubricants, disintegrants, and glidants. Diluents or fillers are agents which can provide bulk and binding properties. Examples of suitable diluents or fillers are lactose, mannitol, sucrose, and corn starch. Typically, such diluents or fillers can be employed in the formulation up to 80 weight percent, and preferably up to 60 weight percent. Binders are agents which can bind the components of the tablets together and are typically employed in a wet granulation process. Examples of suitable binders are acacia, starch, gelatin, and polyvinylpyrrolidinone. Typically, such binders are employed in from 3 to 8 weight percent. Lubricants are agents which can prevent sticking to die walls or punch faces. Examples of suitable lubricants are magnesium stearate, and stearic acid. Typically, such lubricants are employed in an amount from 0.5 to 3.0 weight percent. Disintegrants are agents that enable the tablet to break up at the appropriate time. Examples of suitable distintegrants are corn starch, guar gum, potatoe starch, and alginic acid. Glidants are compositions which can aid powder flow. An example of a suitable glidant is fumed silica. Typically, such glidants are employed in an amount from 0.1 to 3.0 weight percent.

The active ingredient(s), cellulose ether, and optional ingredients are uniformly mixed together in powder form to provide a homogeneous mixture. The mixture is then subjected to compression to provide a solid tablet. Before compressing, the mixture can be subjected to a wet or dry granulation process. The powder or granulated mixture is fed to the die of a tablet press and sufficient pressure is applied to form a solid tablet. Such pressure can vary, and typically ranges from 1,000 to 6,000 psi (7 to 41 MPa), and preferably about 2,000 psi (14 MPa) force. A solid tablet can substantially retain its form under conventional storage and handling conditions. The tablet also maintains its solid form upon administration, and provides sustained release of the active composition through diffusion and erosion.

Advantageously, the ingredients for the pharmaceutical formulation can be treated in a dry granulation process or a wet granulation process. In a dry granulation process, the mixture is precompressed and milled into the desired size prior to tableting. In a wet granulation process, the mixture is combined and formed into granules with a polymeric binder solution and then sized and/or dried at the desired particle size prior to tableting. The size of the granulated mixture is not critical to the active ingredient release rate. The release rate is affected, according to this invention, by the particle size of the cellulose ether prior to granulating.

The tablets are suitable for administering one or more therapeutically active ingredients to humans. Upon contacting the aqueous acidic environment typically present in humans, the tablets slowly dissolve. Typically, the acidic environment is provided by gastric juices, and is at about 37° C.

Solid tablets formulated with the small particle sized cellulose ether composition of this invention surprisingly have a longer release profile compared to tablets formulated with a chemically identical cellulose ether composition which has a larger particle size distribution. When the cellulose ether composition has a particle size sufficiently small that at least 50 weight percent can pass through a 100 mesh (150 micrometers) screen, the tablets typically require at least one hour, preferably at least two hours, and more preferably at least four hours longer to release the active ingredient(s) compared to tablets formulated with a chemically identical cellulose ether composition having a particle size in which less than 50 weight percent can pass through a 100 mesh (150 micrometers) screen.

The following examples are illustrative only, and are not intended to limit the scope of the invention.

### Example 1

The particle size distributions of two samples of HPC are measured and the results are provided in Table A.

**TABLE A**

| Sieve Size mesh (micrometers) | Weight Percent Retained | |
|---|---|---|
| | HPC #1 | HPC C-1* |
| 60 (250) | 28.19 | 56.85 |
| 80 (180) | 12.09 | 15.93 |
| 100 (150) | 9.26 | 6.06 |
| 140 (100) | 3.92 | 8.80 |
| 200 (70) | 4.67 | 5.31 |
| 325 (40) | 31.36 | 4.81 |
| Pan | 10.51 | 2.24 |

| | | |
|---|---|---|
| *Not an example of this invention. | | |

A tablet is formulated using the samples of HPC of Table A. The formulation is:
68.3 percent lactose Fast Flo® (Foremost McKesson)
16.0 percent pseudoephedrine, HCl salt
15 percent HPC polymer
0.7 percent magnesium stearate.
The tablets are 750 mg, and are compressed at 3,000 psi (20.7 MPa) in a 1/2 inch (1.3 cm) standard concave punch. The tablets are dissolved in a USP paddle dissolution device at 100 rpm in a 0.1 N HCl solution at 37° C. The percent dissolved over time is provided in Table B.

**TABLE B**

| Time (Hours) | Percent Dissolved⁽¹⁾ | |
|---|---|---|
| | HPC #1 | HPC C-1* |
| 0 | 0 | 0 |
| 0.5 | 21 | 44 |
| 1.5 | 44 | 96 |
| 3.0 | 64 | 100 |
| 5.0 | 81 | |
| 7.0 | 94 | |
| 9.0 | 98 | |
| 11.0 | 99 | |

| | | |
|---|---|---|
| *Not an example of the invention. | | |
| ⁽¹⁾ Percent of pseudoephedrine, HCl salt dissolved into solution. | | |

This example illustrates the delayed release effect that a fine particle size (at least 50 weight percent passing through 100 mesh (150 micrometers) screen, HPC #1) HPC provides for an active ingredient versus the release from a coarse particle sized HPC (HPC C-1).

### Example 2

Tablets are prepared employing the HPC's of Example 1 in combination with HPMC 2910 and HPMC 2208 The tablet formulation is:
78 percent lactose, spray dried
9 percent HPMC
6 percent HPC
5 percent riboflavin
2 percent stearic acid.

The tablets are 1,000 mg and are compressed at 3,000 psi (20.7 MPa) in a 1/2 inch (1.3 cm) standard concave punch. The dissolution rates of the tablets are measured according to the method of Example 1 and the results are provided in the following table.

| Time (Hours) | HPMC 2910⁽²⁾/HPC-1 | Percent Dissolved HPMC 2910⁽²⁾/HPC C-1* | HPMC 2208⁽³⁾/HPC-1 | HPMC⁽³⁾/HPC 2208 C-1* |
|---|---|---|---|---|
| 0 | 0 | 0 | 0 | 0 |
| 0.5 | 24.6 | 25.8 | 12.7 | 21 |
| 1.5 | 35.8 | 36.4 | 19.3 | 26.4 |
| 3.0 | 46.8 | 44.7 | 26.8 | 39.1 |
| 5.0 | 60.8 | 95.7 | 36.6 | 54.6 |
| 7.0 | 85.8 | 100 | 47.2 | 68.6 |
| 9.0 | Stopped Test | -- | 58.2 | Stopped Test |
| 11.0 | | | 97.9 | |
| 13.0 | | | 100 | |

| | | | | |
|---|---|---|---|---|
| *Not an example of this invention. | | | | |
| ⁽²⁾ Has a hydroxypropoxyl content of between 7 and 12 weight percent, a methoxyl content of between 28 and 30 weight percent, and a 2 percent aqueous solution viscosity of about 4,000 cps. (4 Pa.s) | | | | |
| ⁽³⁾ Has a hydroxypropoxyl content of between 4 and 12 weight percent, a methoxyl content of between 19 and 25 weight percent, and a 2 percent aqueous solution viscosity of about 4,000 cps. (4 Pa.s) | | | | |

This example illustrates the advantages fine particle sized HPC provides to known sustained release polymers.

### Example 3

A course particle sized HPC is ball milled to a finer particle size, and is compared to a coarse HPC in the following table.

| Sieve Size mesh (micrometers) | Weight Percent Retained | |
|---|---|---|
| | HPC-2 (Fine) | HPC C-2* (Coarse) |
| 60 (250) | 20.1 | 76.3 |
| 80 (180) | 15.6 | 9.2 |
| 100 (150) | 11.5 | 3.1 |
| 140 (100) | 30.5 | 4.5 |
| 200 (70) | 2.9 | 0.2 |
| <200 (<70) | 19.6 | 5.1 |

| | | |
|---|---|---|
| *Not an example of this invention. | | |

Tablets are prepared according to the following formulation:
56.67 percent lactose
16 percent pseudoephedrine, HCl salt
26.67 percent HPC
10.67 percent magnesium stearate.
Three tablets are prepared, one using HPC C-2*, one using the 100 to 140 mesh (100 micrometers) fraction of HPC-2, and one using the < 200 mesh (<70 micrometers) fraction of HPC-2.

The dissolution rates of the tablets are measured according to the method of Example 1 and the results are provided in the following Table:

| Time (Hours) | HPC-2 (-100 to -140 mesh) (-150 to -100 micrometers) | Percent Dissolved HPC-2 (<-200 mesh) (<-70 micrometers) | HPC C-2* |
|---|---|---|---|
| 0 | 0 | 0 | 0 |
| 0.5 | 25.17 | 23.4 | 46.47 |
| 1.5 | 42.7 | 44.53 | 90.34 |
| 3.0 | 61.1 | 63.96 | 97.7 |
| 5.0 | 76.5 | 81.73 | 100 |
| 7.0 | 88.2 | 91.7 | |
| 9.0 | 94.7 | 97.3 | |
| 11.0 | 98.7 | 100 | |
| 13.0 | 100 | -- | |

| | | | |
|---|---|---|---|
| *Not an example of this invention. | | | |

## Claims (Claims for the following Contracting State(s): BE, CH, DE, FR, GB, IT, NL, SE)

1. A sustained release solid pharmaceutical formulation for oral administration comprising at least one active ingredient; 5 to 90 weight percent of a polymeric matrix base comprising a water-soluble cellulose ether and, optionally, a hydrophilic colloid; and optionally one or more carriers or excipients, characterised in that said cellulose ether is hydroxypropyl cellulose ether having hydroxypropoxyl substitution of from 20 to 80 weight percent and of a fine particle size whereby at least 50 weight percent of said cellulose ether particles can pass through a 150 micrometers (100 mesh) screen.

2. A formulation as claimed in Claim 1 in a tablet form.

3. A formulation as claimed in Claim 1 or Claim 2, wherein said hydroxypropoxyl substitution is from 40 to 80 weight percent.

4. A formulation as claimed in any one of the preceding claims, wherein the amount of hydroxypropyl cellulose ether is from 10 to 17 weight percent.

5. A formulation as claimed in any one of the preceding claims, wherein at least 70 weight percent of said cellulose ether particles can pass through said screen.

6. A formulation as claimed in any one of the preceding claims, wherein said hydrophilic colloid is present.

7. A formulation as claimed in Claim 6, wherein said hydrophilic colloid is hydroxypropyl methylcellulose ether having a hydroxypropoxyl content of between 4 and 12 weight percent, a methoxyl content of between 19 and 30 weight percent, and a 2 percent aqueous solution viscosity of between 0.1 and 100 Pa.s (100 and 100,000 cps).

8. A formulation as claimed in Claim 7, wherein the hydroxypropyl methylcellulose ether is present in an amount of from 5 to 75 weight percent of the combined weight of the hydroxypropyl cellulose and hydroxypropyl methyl-cellulose ethers.

9. A formulation as claimed in Claim 7 or Claim 8, wherein the hydroxypropyl cellulose ether comprises at least 30 weight percent of the combined weight of the hydroxypropyl cellulose ether and the hydroxypropyl methylcellulose ether.

10. A formulation as claimed in Claim 9, wherein at least 70 weight percent of the hydroxypropyl methylcellulose ether particles can pass through a 150 micrometers (100 mesh) screen.

11. A formulation as claimed in Claim 10, wherein at least 70 weight percent of the hydroxypropyl methylcellulose ether particles can pass through a 100 micrometers (140 mesh) screen.

12. A formulation as claimed in any one of the preceding claims, wherein the hydroxypropyl cellulose ether is the reaction product of reacting propylene oxide with alkali cellulose.

13. The use, optionally with a hydrophilic colloid, of a water-soluble hydroxypropyl cellulose ether having hydroxypropoxyl substitution of from 20 to 80 weight percent and of a fine particle size whereby at least 50 weight percent of said cellulose ether particles can pass through a 150 micrometers (100 mesh) screen as a polymeric matrix base of a sustained release solid pharmaceutical formulation for oral administration comprising at least one active ingredient to further delay the release of said active ingredient therefrom compared with otherwise identical hydroxypropyl cellulose ether of larger particle size.

14. A use as claimed in Claim 13, wherein said hydroxypropyl cellulose ether and/or said hydrophilic colloid and/or the amounts thereof are as specified in any one of Claims 3 to 12.

## Claims (Claims for the following Contracting State(s): AT)

1. A process for preparing a sustained release solid pharmaceutical formulation for oral administration which comprises mixing together, in powder form at least one active ingredient; 5 to 90 weight percent of a polymeric matrix base comprising a water-soluble cellulose ether and, optionally, a hydrophilic colloid; and optionally one or more carriers or excipients, to provide a homogeneous mixture and forming said mixture into a solid form, characterised in that said cellulose ether is hydroxypropyl cellulose ether having hydroxypropoxyl substitution of from 20 to 80 weight percent and of a fine particle size whereby at least 50 weight percent of the cellulose ether particles can pass through a 150 micrometers (100 mesh) screen.

2. A process as claimed in Claim 1 in a tablet form.

3. A process as claimed in Claim 1 or Claim 2, wherein said hydroxypropoxyl substitution is from 40 to 80 weight percent.

4. A process as claimed in any one of the preceding claims, wherein the amount of hydroxypropyl cellulose ether is from 10 to 17 weight percent.

5. A process as claimed in any one of the preceding claims, wherein at least 70 weight percent of said cellulose ether particles can pass through said screen.

6. A process as claimed in any one of the preceding claims, wherein said hydrophilic colloid is present.

7. A process as claimed in Claim 6, wherein said hydrophilic colloid is hydroxypropyl methylcellulose ether having a hydroxypropoxyl content of between 4 and 12 weight percent, a methoxyl content of between 19 and 30 weight percent, and a 2 percent aqueous solution viscosity of between 0.1 and 100 Pa.s (100 and 100,000 cps).

8. A process as claimed in Claim 7, wherein the hydroxypropyl methylcellulose ether is present in an amount of from 5 to 75 weight percent of the combined weight of the hydroxypropyl cellulose and hydroxypropyl methyl-cellulose ethers.

9. A process as claimed in Claim 7 or Claim 8, wherein the hydroxypropyl cellulose ether comprises at least 30 weight percent of the combined weight of the hydroxypropyl cellulose ether and the hydroxypropyl methylcellulose ether.

10. A process as claimed in Claim 9, wherein at least 70 weight percent of the hydroxypropyl methylcellulose ether particles can pass through a 150 micrometers (100 mesh) screen.

11. A process as claimed in Claim 9, wherein at least 70 weight percent of the hydroxypropyl methylcellulose ether particles can pass through a 100 micrometers (140 mesh) screen.

12. A process as claimed in any one of the preceding claims, wherein the hydroxypropyl cellulose ether is the reaction product of reacting propylene oxide with alkali cellulose.

13. The use, optionally with a hydrophilic colloid, of a water-soluble hydroxypropyl cellulose ether having hydroxypropoxyl substitution of from 20 to 80 weight percent and of a fine particle size whereby at least 50 weight percent of said cellulose ether particles can pass through a 150 micrometers (100 mesh) screen as a polymeric matrix base of a sustained release solid pharmaceutical formulation for oral administration comprising at least one active ingredient to further delay the release of said active ingredient therefrom compared with otherwise identical hydroxypropyl cellulose ether of larger particle size.

14. A use as claimed in Claim 13, wherein said hydroxypropyl cellulose ether and/or said hydrophilic colloid and/or the amounts thereof are as specified in any one of Claims 3 to 12.

## Patentansprüche (Patentansprüche für folgende(n) Vertragsstaat(en): BE, CH, DE, FR, GB, IT, NL, SE)

1. Feste pharmazeutische Formulierung zur anhaltenden Freisetzung zur oralen Verabreichung, umfassend mindestens einen Wirkstoff, 5 bis 90 Gew.-% einer polymeren Grundmatrix, die einen wasserlöslichen Celluloseether und gegebenenfalls ein hydrophiles Kolloid umfaßt, und gegebenenfalls ein oder mehrere Trägermittel oder Vehikel, dadurch gekennzeichnet, daß der Celluloseether Hydroxypropylcelluloseether mit einer Hydroxypropoxyl-Substitution von 20 bis 80 Gew.-% und einer feinen Teilchengröße ist, so daß mindestens 50 Gew.-% der Celluloseether-Teilchen ein 150 Mikrometer (100 mesh) Sieb passieren können.

2. Formulierung nach Anspruch 1 in Tablettenform.

3. Formulierung nach Anspruch 1 oder 2, worin die Hydroxypropoxyl-Substitution von 40 bis 80 Gew.-% beträgt.

4. Formulierung nach einem der vorhergehenden Ansprüche, worin die Menge an Hydroxypropylcelluloseether von 10 bis 17 Gew.-% beträgt.

5. Formulierung nach einem der vorhergehenden Ansprüche, worin mindestens 70 Gew.-% der Celluloseether-Teilchen das Sieb passieren können.

6. Formulierung nach einem der vorhergehenden Ansprüche, worin das hydrophile Kolloid vorhanden ist.

7. Formulierung nach Anspruch 6, worin das hydrophile Kolloid Hydroxypropylmethylcelluloseether mit einem Hydroxypropoxylgehalt zwischen 4 und 12 Gew.-%, einem Methoxylgehalt zwischen 19 und 30 Gew.-% und einer Viskosität in 2 %-iger wäßriger Lösung zwischen 0,1 und 100 Pa· s (100 und 100.000 cps) ist.

8. Formulierung nach Anspruch 7, worin der Hydroxypropylmethyl- celluloseether in einer Menge von 5 bis 75 Gew.-% des Gesamtgewichts des Hydroxypropylcellulose- und Hydroxypropylmethylcelluloseethers vorhanden ist.

9. Formulierung nach Anspruch 7 oder 8, worin der Hydroxypropylcelluloseether mindestens 30 Gew.-% des Gesamtgewichts des Hydroxypropylcelluloseethers und des Hydroxypropylmethylcelluloseethers umfaßt.

10. Formulierung nach Anspruch 9, worin mindestens 70 Gew.-% der Hydroxypropylmethylcelluloseether-Teilchen ein 150 Mikrometer (100 mesh) Sieb passieren können.

11. Formulierung nach Anspruch 10, worin mindestens 70 Gew.-% der Hydroxypropylmethylcelluloseether-Teilchen ein 100 Mikrometer (140 mesh) Sieb passieren können.

12. Formulierung nach einem der vorhergehenden Ansprüche, worin der Hydroxypropylcelluloseether das Reaktionsprodukt einer Reaktion von Propylenoxid mit Alkalicellulose ist.

13. Verwendung eines wasserlöslichen Hydroxypropylcelluloseethers mit einer Hydroxypropoxyl-Substitution von 20 bis 80 Gew.-% und einer feinen Teilchengröße, so daß mindestens 50 Gew.-% der Celluloseether-Teilchen ein 150 Mikrometer (100 mesh) Sieb passieren können, gegebenenfalls mit einem hydrophilen Kolloid als polymere Grundmatrix einer festen pharmazeutischen, mindestens einen Wirkstoff umfassenden Formulierung zur anhaltenden Freisetzung zur oralen Verabreichung, um die Freisetzung des Wirkstoffes daraus verglichen mit einem ansonsten identischen Hydroxypropylcelluloseether einer höheren Teilchengröße weiter zu verzögern.

14. Verwendung nach Anspruch 13, worin der Hydroxypropylcelluloseether und/oder das hydrophile Kolloid und/oder deren Mengen wie in einem der Ansprüche 3 bis 12 angegeben sind.

## Patentansprüche (Patentansprüche für folgende(n) Vertragsstaat(en): AT)

1. Verfahren zur Herstellung einer festen pharmazeutischen Formulierung zur anhaltenden Freisetzung zur oralen Verabreichung, umfassend das Vermischen von mindestens einem Wirkstoff in Pulverform, 5 bis 90 Gew.-% einer polymeren Grundmatrix, die einen wasserlöslichen Celluloseether und gegebenenfalls ein hydrophiles Kolloid umfaßt, und gegebenenfalls einem oder mehreren Trägermitteln oder Vehikeln, um ein homogenes Gemisch zu erhalten, und Bringen des Gemisches in eine feste Form, dadurch gekennzeichnet, daß der Celluloseether Hydroxypropylcelluloseether mit einer Hydroxypropoxyl-Substitution von 20 bis 80 Gew.-% und einer feinen Teilchengröße ist, so daß mindestens 50 Gew.-% der Celluloseether-Teilchen ein 150 Mikrometer (100 mesh) Sieb passieren können.

2. Verfahren nach Anspruch 1 in Tablettenform.

3. Verfahren nach Anspruch 1 oder 2, worin die Hydroxypropoxyl-Substitution von 40 bis 80 Gew.-% beträgt.

4. Verfahren nach einem der vorhergehenden Ansprüche, worin die Menge an Hydroxypropylcelluloseether von 10 bis 17 Gew.-% beträgt.

5. Verfahren nach einem der vorhergehenden Ansprüche, worin mindestens 70 Gew -% der Celluloseether-Teilchen das Sieb passieren können.

6. Verfahren nach einem der vorhergehenden Ansprüche, worin das hydrophile Kolloid vorhanden ist.

7. Verfahren nach Anspruch 6, worin das hydrophile Kolloid Hydroxypropylmethylcelluloseether mit einem Hydroxypropoxylgehalt zwischen 4 und 12 Gew.-%, einem Methoxylgehalt zwischen 19 und 30 Gew.-% und einer Viskosität in 2 %-iger wäßriger Lösung zwischen 0,1 und 100 Pa·s (100 und 100.000 cps) ist.

8. Verfahren nach Anspruch 7, worin der Hydroxypropylmethylcelluloseether in einer Menge von 5 bis 75 Gew.-% des Gesamtgewichts des Hydroxypropylcellulose- und Hydroxypropylmethylcelluloseethers vorhanden ist.

9. Verfahren nach Anspruch 7 oder 8, worin der Hydroxypropylcelluloseether mindestens 30 Gew.-% des Gesamtgewichts des Hydroxypropylcelluloseethers und des Hydroxypropylmethylcelluloseethers umfaßt.

10. Verfahren nach Anspruch 9, worin mindestens 70 Gew.-% der Hydroxypropylmethylcelluloseether-Teilchen ein 150 Mikrometer (100 mesh) Sieb passieren können.

11. Verfahren nach Anspruch 9, worin mindestens 70 Gew.-% der Hydroxypropylmethylcelluloseether-Teilchen ein 100 Mikrometer (140 mesh) Sieb passieren können.

12. Verfahren nach einem der vorhergehenden Ansprüche, worin der Hydroxypropylcelluloseether das Reaktionsprodukt einer Reaktion von Propylenoxid mit Alkalicellulose ist.

13. Verwendung eines wasserlöslichen Hydroxypropylcelluloseethers mit einer Hydroxypropoxyl-Substitution von 20 bis 80 Gew.-% und einer feinen Teilchengröße, so daß mindestens 50 Gew.-% der Celluloseether-Teilchen ein 150 Mikrometer (100 mesh) Sieb passieren können, gegebenenfalls mit einem hydrophilen Kolloid, als polymere Grundmatrix einer festen pharmazeutischen, mindestens einen Wirkstoff enthaltenden Formulierung zur anhaltenden Freisetzung zur oralen Verabreichung, um die Freisetzung des Wirkstoffes daraus verglichen mit einem ansonsten identischen Hydroxypropylcelluloseether einer höheren Teilchengröße weiter zu verzögern.

14. Verwendung nach Anspruch 13, worin der Hydroxypropylcelluloseether und/oder das hydrophile Kolloid und/oder deren Mengen wie in einem der Ansprüche 3 bis 12 angegeben sind.

## Revendications (Revendications pour l'(les) Etat(s) contractant(s) suivant(s): BE, CH, DE, FR, GB, IT, NL, SE)

1. Formulation pharmaceutique solide à libération prolongée, destinée à être administrée par voie orale, comprenant au moins un ingrédient actif, de 5 à 90 % en poids d'une matrice-base polymère comprenant un éther hydrosoluble de cellulose et, éventuellement, un colloïde hydrophile et, éventuellement, un ou plusieurs véhicules ou excipients, caractérisée en ce que ledit éther de cellulose est un éther hydroxypropyl-cellulose dont le degré de substitution hydroxypropoxylique vaut de 20 à 80 % en poids et dont la granulométrie est fine, au point qu'au moins 50 % en poids des particules de cet éther de cellulose peuvent passer à travers un tamis de 150 µm (100 mesh).

2. Formulation conforme à la revendication 1, sous forme de comprimés.

3. Formulation conforme à la revendication 1 ou 2, dans laquelle le degré de substitution hydroxypropoxylique vaut de 40 à 80 % en poids.

4. Formulation conforme à l'une quelconque des revendications précédentes, dans laquelle la proportion d'éther hydroxypropylcellulose vaut de 10 à 17 % en poids.

5. Formulation conforme à l'une quelconque des revendications précédentes, dans laquelle au moins 70 % en poids des particules de l'éther de cellulose peuvent passer à travers ledit tamis.

6. Formulation conforme à l'une quelconque des revendications précédentes, dans laquelle ledit colloïde hydrophile est présent.

7. Formulation conforme à la revendication 6, dans laquelle ledit colloïde hydrophile est un éther hydroxypropyl-méthyl-cellulose, dont la teneur en groupes hydroxypropoxy vaut entre 4 et 12 % en poids, la teneur en groupes méthoxy vaut entre 19 et 30 % en poids, et la viscosité, en solution aqueuse à 2 %, vaut entre 0,1 et 100 Pa.s (entre 100 et 100 000 cps).

8. Formulation conforme à la revendication 7, dans laquelle l'éther hydroxypropyl-méthyl-cellulose se trouve en une proportion de 5 à 75 % en poids, par rapport au poids combiné des éthers hydroxypropyl-cellulose et hydroxypropyl-méthyl-cellulose.

9. Formulation conforme à la revendication 7 ou 8, dans laquelle le poids de l'éther hydroxypropyl-cellulose représente au moins 30 % du poids combiné des éthers hydroxypropyl-cellulose et hydroxypropyl-méthyl-cellulose.

10. Formulation conforme à la revendication 9, dans laquelle au moins 70 % en poids des particules d'éther hydroxypropyl-méthyl-cellulose peuvent passer à travers un tamis de 150 µm (100 mesh).

11. Formulation conforme à la revendication 10, dans laquelle au moins 70 % en poids des particules d'éther hydroxypropyl-méthyl-cellulose peuvent passer à travers un tamis de 100 µm (140 mesh).

12. Formulation conforme à l'une quelconque des revendications précédentes, dans laquelle l'éther hydroxypropyl-méthyl-cellulose est un produit de réaction d'oxyde de propylène avec une alcali-cellulose.

13. Utilisation, éventuellement avec un colloïde hydrophile, d'un éther hydroxypropyl-cellulose dont le degré de substitution hydroxypropoxylique vaut de 20 à 80 % en poids et dont la granulométrie est fine, au point qu'au moins 50 % en poids des particules de cet éther de cellulose peuvent passer à travers un tamis de 150 µm (100 mesh), comme matrice-base polymère d'une formulation pharmaceutique solide à libération prolongée, destinée à être administrée par voie orale, contenant au moins un ingrédient actif, pour retarder davantage la libération dudit ingrédient actif de cette formulation, en comparaison d'un éther hydroxypropyl-cellulose de granulométrie plus grosse, mais identique par ailleurs.

14. Utilisation conforme à la revendication 13, pour laquelle ledit éther hydroxypropyl-cellulose et/ou ledit colloïde hydrophile et/ou leurs proportions sont tels que définis dans l'une quelconque des revendications 3 à 12.

## Revendications (Revendications pour l'(les) Etat(s) contractant(s) suivant(s): AT)

1. Procédé de préparation d'une formulation pharmaceutique solide à libération prolongée, destinée à être administrée par voie orale, comprenant le fait de mélanger au moins un ingrédient actif, de 5 à 90 % en poids d'une matrice-base polymère comprenant un éther hydrosoluble de cellulose et, éventuellement, un colloïde hydrophile et, éventuellement, un ou plusieurs véhicules ou excipients, pour obtenir un mélange homogène, et le fait de mettre ce mélange sous une forme solide, caractérisé en ce que ledit éther de cellulose est un éther hydroxypropyl-cellulose dont le degré de substitution hydroxypropoxylique vaut de 20 à 80 % en poids et dont la granulométrie est fine, au point qu'au moins 50 % en poids des particules de cet éther de cellulose peuvent passer à travers un tamis de 150 µm (100 mesh).

2. Procédé conforme à la revendication 1, pour former des comprimés.

3. Procédé conforme à la revendication 1 ou 2, dans lequel ledit degré de substitution hydroxypropoxylique vaut de 40 à 80 % en poids.

4. Procédé conforme à l'une quelconque des revendications précédentes, dans lequel la proportion d'éther hydroxypropyl-cellulose vaut de 10 à 17 % en poids.

5. Procédé conforme à l'une quelconque des revendications précédentes, dans lequel au moins 70 % en poids des particules de l'éther de cellulose peuvent passer à travers ledit tamis.

6. Procédé conforme à l'une quelconque des revendications précédentes, dans lequel ledit colloïde hydrophile est présent.

7. Procédé conforme à la revendication 6, dans lequel ledit colloïde hydrophile est un éther hydroxypropyl-méthyl-cellulose, dont la teneur en groupes hydroxypropoxy vaut entre 4 et 12 % en poids, la teneur en groupes méthoxy vaut entre 19 et 30 % en poids, et la viscosité, en solution aqueuse à 2 %, vaut entre 0,1 et 100 Pa.s (entre 100 et 100 000 cps).

8. Procédé conforme à la revendication 7, dans lequel l'éther hydroxypropyl-méthyl-cellulose se trouve en une proportion de 5 à 75 % en poids, par rapport au poids combiné des éthers hydroxypropyl-cellulose et hydroxypropyl-méthyl-cellulose.

9. Procédé conforme à la revendication 7 ou 8, dans lequel le poids de l'éther hydroxypropyl-cellulose représente au moins 30 % du poids combiné des éthers hydroxypropyl-cellulose et hydroxypropyl-méthyl-cellulose.

10. Procédé conforme à la revendication 9, dans lequel au moins 70 % en poids des particules d'éther hydroxypropyl-méthyl-cellulose peuvent passer à travers un tamis de 150 µm (100 mesh).

11. Procédé conforme à la revendication 10, dans lequel au moins 70 % en poids des particules d'éther hydroxypropyl-méthyl-cellulose peuvent passer à travers un tamis de 100 µm (140 mesh).

12. Procédé conforme à l'une quelconque des revendications précédentes, dans lequel l'éther hydroxypropyl-méthyl-cellulose est un produit de réaction d'oxyde de propylène avec une alcali-cellulose.

13. Utilisation, éventuellement avec un colloïde hydrophile, d'un éther hydroxypropyl-cellulose dont le degré de substitution hydroxypropoxylique vaut de 20 à 80 % en poids et dont la granulométrie est fine, au point qu'au moins 50 % en poids des particules de cet éther de cellulose peuvent passer à travers un tamis de 150 µm (100 mesh), comme matrice-base polymère d'une formulation pharmaceutique solide à libération prolongée, destinée à être administrée par voie orale, contenant au moins un ingrédient actif, pour retarder davantage la libération dudit ingrédient actif de cette formulation, en comparaison d'un éther hydroxypropyl-cellulose de granulométrie plus grosse, mais identique par ailleurs.

14. Utitisation conforme à la revendication 13, pour laquelle ledit éther hydroxypropylcellulose et/ou ledit colloïde hydrophile et/ou leurs proportions sont tels que définis dans l'une quelconque des revendications 3 à 12.
